# EUROPEAN PATENT APPLICATION

(11) **EP 4 483 861 A1**
(43) Date of publication of application: **01.01.2025**
(21) Application number: 23182492.1
(22) Date of filing: 29.06.2023
(51) Int. Cl.: A61K 8/19, A61K 8/24, A61K 8/25, A61K 8/60, A61Q 11/00

(54) **ORAL CARE COMPOSITION COMPRISING ALKYL POLYGLUCOSIDE**

(71) Applicant: Unilever IP Holdings B.V., 3013 AL Rotterdam (NL)
(72) Inventor: BARILI, Matteo, 6708 WH Wageningen (NL); VACCHELLI, Valeria, 6708 WH Wageningen (NL)
(74) Representative: Tansley, Sally Elizabeth

(57) **Abstract**

A toothpaste composition comprising:
a) a silica based abrasive;
b) an alkyl polyglucoside surfactant; and
c) a buffer selected from trisodium phosphate, sodium bicarbonate, monosodium phosphate, potassium citrate or mixtures thereof.

## Description

### Technical Field of the invention

The present invention relates to oral care compositions such as toothpastes, powders, gums, mouthwashes and the like. In particular the present invention relates to an oral care compositions, particularly toothpastes that are effective in mitigating the effects of enamel demineralisation.

### Background of the invention

Oral care compositions comprising alkyl poly glucosides are disclosed in EP3883540 and US11633337.

There remains the need for a toothpaste composition that comprises mild surfactants yet mitigates the effects of enamel demineralisation.

### Description of the invention

The present invention relates to a toothpaste composition comprising:
a) a silica based abrasive;
b) an alkyl polyglucoside surfactant; and
c) a buffer selected from trisodium phosphate, sodium bicarbonate, monosodium phosphate, potassium citrate or mixtures thereof

### Detailed Description

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any ranges of values, any upper value can be associated with any particular lower value.

Where a feature is disclosed with respect to a particular aspect of the invention (for example a composition of the invention), such disclosure is also to be considered to apply to any other aspect of the invention (for example a method of the invention) *mutatis mutandis.*

Any ingredients mentioned in this application that are natural or naturally derived have been sourced from Europe.

In the context of the present invention the term substantially free means comprising less than 0.01wt% of the total composition of the excluded ingredient.

The composition of the invention is used to preferably used to clean the surfaces of the oral cavity.

Preferably the toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions according to the invention comprise one or more alkyl polyglucosides, such as one or more C6-C18 alkyl polyglucosides, or the like. The C6-C18 alkyl polyglucosides may include one or more short chain alkyl polyglucosides, one or more long chain alkyl polyglucosides, or any combination thereof. In the contecxt of the present invention, the term "short chain alkyl polyglucosides" refers to alkyl polyglucosides having a chain length of from about 6 to about 10 carbon atoms. As used herein, the term or expression "long chain alkyl polyglucosides" may refer to alkyl polyglucosides having a chain length of from about 11 to about 18 carbon atoms. It should be appreciated that the alkyl polyglucosides may include a hydrophobic fatty alcohol portion and a hydrophilic glucoside portion. In a preferred implementation, the nonionic surfactants include at least a short chain alkyl polyglucoside, such as a C6-C10 alkyl polyglucoside or a C8-C10 alkyl polyglucoside, decyl glucoside is particularly preferred, The oral care composition is free or substantially free of long chain alkyl polyglucosides.

The level of alkylpolyglucoside in the toothpaste composition according is preferably from 0.1 to 1.5 wt%, more preferably from 0.5 to 1 wt% of the total composition.

The composition is substantially free of anionic surfactants such as alkyl sulphate salts.

Compositions according to the invention comprise a pH buffering agent selected from sodium phosphate, sodium bicarbonate, monosodium phosphate, potassium citrate or mixtures thereof

The pH buffering agent/agents are present in a total amount from 0.1 to 1.5 wt% of the total composition.

The pH of the composition at 20°C is from 7.5 to 9.2, preferably from 7.8 to 8.4.

Compositions according to the invention, comprise particulate abrasive silica material, preferably in amounts between 3 and 60% by weight of the oral care composition.

The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

The composition, preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Further suitable binders and thickeners can be present and include as sodium carboxymethylcellulose, hydroxyethyl cellulose (Natrosol^{®}), xanthan gum, gum arabic etc. as well as synthetic polymers such as polyacrylates and carboxyvinyl polymers such as Carbopol^{®}.

Compositions according to the invention may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.
Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise water-soluble or sparingly water-soluble sources of metal salts Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprises fluoride sources such as sodium fluoride, stannous fluoride, sodium monofluorophosphate, zinc ammonium fluoride, tin ammonium fluoride, calcium fluoride, cobalt ammonium fluoride and mixtures thereof. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. In some embodiment, the stannous fluoride is present in an amount of 0.1 wt. percent to 2 wt. percent (0.1 wt percent -0.6 wt. percent) of the total composition weight. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. However, it is to be understood that the weights of fluoride salts to provide the appropriate level of fluoride ion will obviously vary based on the weight of the counter ion in the salt, and one of skill in the art may readily determine such amounts. In some embodiment, the fluoride source is a fluoride salt present in an amount of 0.1 wt. percent to 2 wt. percent (0.1 wt percent -0.6 wt. percent) of the total composition weight (e.g., sodium fluoride (e.g., about 0.32 wt. percent). Some embodiments of the invention are free from fluoride sources, that is the composition comprises less than 0.01 wt% of a fluoride source.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

In some embodiments, the oral care compositions comprise an effective amount of one or more antibacterial agents, for example comprising an antibacterial agent selected from halogenated diphenyl ether (e.g. triclosan), triclosan monophosphate, herbal extracts and essential oils (e.g., rosemary extract, tea extract, magnolia extract, thymol, menthol, eucalyptol, geraniol, carvacrol, citral, hinokitol, magnolol, ursolic acid, ursic acid, catechol, methyl salicylate, epigallocatechin gallate, epigallocatechin, gallic acid, miswak extract, sea-buckthom extract), bisguanide antiseptics (e.g., chlorhexidine, alexidine or octenidine), quaternary ammonium compounds (e.g., cetylpyridinium chloride (CPC), CPC-claybenzalkonium chloride, tetradecylpyridinium chloride (TPC), N-tetradecyl-4-ethylpyridinium chloride (TDEPC)), phenolic antiseptics, hexetidine furanones, bacteriocins, ethyllauroyl arginate, arginine bicarbonate, a Camellia extract, a flavonoid, a flavan, halogenated diphenyl ether, creatine, sanguinarine, povidone iodine, delmopinol, salifluor, metal ions (e.g., zinc salts, stannous salts, copper salts, iron salts), propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nisin preparations, chlorite salts; parabens such as methylparaben or propylparaben and mixtures of any of the foregoing. One or more additional antibacterial or preservative agents may optionally be present in the composition in a total amount of from about 0.01 wt. percent to about 0.5 wt. percent, optionally about 0.05 wt. percent to about 0.1 wt. percent or about 0.3 percent, by total weight of the composition.

In some embodiments, the oral care compositions also comprise at least one flavorant, useful for example to enhance taste of the composition. Any orally acceptable natural or synthetic flavorant can be used, including without limitation essential oils and various flavoring aldehydes, esters, alcohols, and similar materials, tea flavors, vanillin, sage, marjoram, parsley oil, spearmint oil, cinnamon oil, oil of wintergreen, peppermint oil, clove oil, bay oil, anise oil, eucalyptus oil, citrus oils, fruit oils, sassafras and essences including those derived from lemon, orange, lime, grapefruit, apricot, banana, grape, apple, strawberry, cherry, pineapple, etc., bean-and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, carvone, menthyl acetate, menthyl lactate, camphor, eucalyptus oil, eucalyptol, anethole, eugenol, cassia, oxanone, a-irisone, , thymol, linalool, benzaldehyde, cinnamaldehyde, N-ethyl-p-menthan-3-carboxamine, N, 2, 3-trimethyl-2- isopropylbutanamide, 3- (1-menthoxy) - propane-1, 2-diol, cinnamaldehyde glycerol acetal (CGA), menthone glycerol acetal (MGA) and the like. One or more flavorants are optionally present in a total amount of from about 0.01 wt. percent to about 5 wt. percent, for example, from about 0.03 wt. percent to about 2.5 wt. percent, optionally about 0.05 wt. percent to about 1.5 wt. percent, further optionally about 0.1 wt. percent to about 0.3 wt. percent and in some embodiments in various embodiments from about 0.01 wt. percent to about 1 wt. percent, from about 0.05 to about 2 percent, from about 0.1 percent to about 2.5 percent, and from about 0.1 to about 0.5 percent by total weight of the composition.

In some embodiments, the oral care compositions comprise at least one sweetener, useful for example to enhance taste of the composition. Sweetening agents among those useful herein include dextrose, polydextrose, sucrose, maltose, dextrin, dried invert sugar, mannose, xylose, ribose, fructose, levulose, galactose, com syrup, partially hydrolyzed starch, hydrogenated starch hydrolysate, ethanol, sorbitol, mannitol, xylitol, maltitol, isomalt, aspartame, neotame, saccharin and salts thereof (e.g. sodium saccharin), sucralose, dipeptide-based intense sweeteners, cyclamates, dihydrochalcones.

One or more sweeteners are optionally present in a total amount depending strongly on the particular sweetener (s) selected, but typically 0.005 wt. percent to 5 wt. percent, by total weight of the composition, optionally 0.005 wt. percent to 0.2 wt. percent, further optionally 0.05 wt. percent to 0.1 wt. percent by total weight of the composition.

In some embodiments, the oral care compositions further comprise an agent that interferes with or prevents bacterial attachment, e.g., ethyl lauroyl arginiate (ELA), solbrol or chitosan, as well as plaque dispersing agents such as enzymes (papain, glucoamylase, etc.).

Mixtures of any of the above described materials may also be used.

The composition according the invention will comprise further ingredients which are common in the art, such as:
antimicrobial agents, e.g. chlorhexidine, sanguinarine extract, metronidazole, quaternary ammonium compounds, such as cetylpyridinium chloride; cetylpyridium chloride clay complex bisguanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C, D, B (preferably B3) and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
mouth feel agents
pharmaceutically acceptable carriers, e.g. starch, sucrose, water or water/alcohol systems etc.; Humectants such as glycerol, sorbitol, propyleneglycol, xylitol, lactitol etc.;
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

### Examples

| **Ingredients** | **Example, wt%** |
|---|---|
| Decyl glucoside | 0.75 |
| Sorbitol (70wt%) | 55 |
| Sodium Fluoride | 0.19 |
| Sodium Monofluorophosphate | 0.11 |
| Hydrated Silica Medium Abrasivity | 4 |
| Hydrated Silica Thickening | 13.5 |
| Sodium carboxy methyl cellulose | 0.7 |
| Flavour | 0.4 |

## Claims

1. A toothpaste composition comprising:
a) a silica based abrasive;
b) an alkyl polyglucoside surfactant; and
c) a buffer selected from trisodium phosphate, sodium bicarbonate, monosodium phosphate, potassium citrate or mixtures thereof.

2. A toothpaste composition according to claim 1 in which the oral care composition is substantially free of alkyl sulphate salts.

3. A toothpaste composition according to any preceding claim in which the pH at 20°C is from 7.5 to 9.2, preferably form 7.8 to 8.4.

4. A toothpaste composition according to any preceding claim in which the alkyl polyglucosde is a C8 6o C10 alkyl polyglucoside, preferably decyl glucoside.

5. A toothpaste composition according to any preceding claim in which the level of (c) pH buffering agent is from 0.1 to 1.5 wt% of the total composition.

6. A toothpaste composition according to any preceding claim in which the level of alkylpolyglucoside is from 0.1 to 1.5 wt%, preferably from 0.5 to 1 wt% of the total composition.

7. A toothpaste composition according to any preceding claim which further comprises a thickening agent, preferably carboxy methyl cellulose.
